(19) Europäisches Patentamt
European Patent Office
Office européen des brevets

(11) Publication number: **0 211 665 B1**

(12) **EUROPEAN PATENT SPECIFICATION**

(45) Date of publication of patent specification: **12.06.91**

(51) Int. Cl.[5]: **C07C 259/00**, C07D 495/08, C07D 339/08, A01N 47/34, //(C07D495/08,335:00,335:00)

(21) Application number: **86306123.0**

(22) Date of filing: **07.08.86**

The file contains technical information submitted after the application was filed and not included in this specification

(54) **Benzoyl-ureas having acaricide and insecticide activity.**

(30) Priority: **08.08.85 IT 2188685**

(43) Date of publication of application: **25.02.87 Bulletin 87/09**

(45) Publication of the grant of the patent: **12.06.91 Bulletin 91/24**

(84) Designated Contracting States: **AT BE CH DE FR GB LI NL SE**

(56) References cited: **EP-A- 0 117 320**

(73) Proprietor: **ISTITUTO GUIDO DONEGANI S.p.A.**
**Via Caduti del Lavoro**
**I-28100 Novara(IT)**

(72) Inventor: **Bianchi, Daniele**
**50, via Mose Bianchi**
**I-20149 Milan(IT)**
Inventor: **Massardo, Pietro**
**5, via Fra Bartolomeo**
**I-20100 Milan(IT)**
Inventor: **Meazza, Giovanni**
**30, via Pastore**
**I-21047 Saronno Varese(IT)**
Inventor: **Rama, Franco**
**1, via Rodi**
**I-21052 Busto Arsizio Varese(IT)**
Inventor: **Caprioli, Vincenzo**
**8, via Loriga**
**I-28028 S. Martino Siccomario Pavia(IT)**

(74) Representative: **Whalley, Kevin et al**
**MARKS & CLERK 57/60 Lincoln's Inn Fields**
**London WC2A 3LS(GB)**

EP 0 211 665 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to benzoyl-ureas having acaricide and insecticide activity, and especially to derivatives of 1-benzoyl-3-aryl-urea particularly active against eggs and larvae of noxious insects and acari in the agrarian and veterinary fields, and to their use. The invention also relates to a process for the preparation of such ureas.

Several derivatives of 1-benzoyl-3-aryl-urea, having insecticide activity are known. Among them, the first product introduced on the market was Diflubenzuron, the usual name of the compound 1-(2,6-difluorobenzoyl)-3-(4-chlorophenyl)-urea, disclosed in U.S. Patent No. US-A-3,993,908 (U.S. Philips Corporation). Diflubenzuron is, however, suspected to be a carcinogen, because it contains the 4-chloro-aniline unit in its molecule [European Chem. News 6 (16), 29 (1978)].

In Chemical Abstract 91, 20141 (1979), benzoylureas are described having both acaricide and insecticide activity such as 1-(2,6-difluorobenzoyl)-3-(4-benzyloxyphenyl)-urea, which, however, at the commonly used doses, has only a marginal acaricide activity.

EP-A-117320 discloses benzoylurea derivatives having insecticide and acaricide activity.

The present invention provides derivatives of 1-benzoyl-3-aryl-urea, having the general formula:

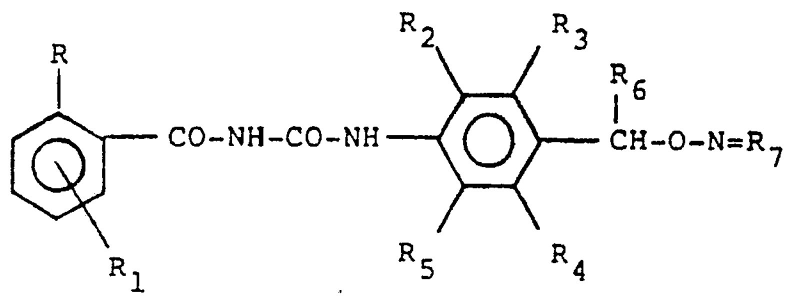

(I)

wherein:

R represents a chlorine or fluorine atom;

$R_1$ represents a hydrogen, chlorine, or fluorine atom;

$R_2$ and $R_5$, which may be the same as or different from each other, represent a hydrogen or halogen atom, or an alkyl group containing from 1 to 4 carbon atoms;

$R_3$ and $R_4$, which may be the same as or different from each other, represent a hydrogen or halogen atom, or an alkyl, haloalkyl, alkoxy, haloalkoxy, alkenyl, haloalkenyl, alkenyloxy, haloalkenyloxy or alkynyl group;

$R_6$ represents a hydrogen atom, or an alkyl or haloalkyl group containing from 1 to 3 carbon atoms; and

$R_7$ represents a monocyclo-, bicyclo- or polycyclo-alkyl or -alkenyl group containing from 5 to 10 carbon atoms, or a monocyclo-, bicyclo-or polycyclo-alkyl or -alkenyl group containing from 4 to 8 carbon atoms and 1 or 2 heteroatoms such as N, S, O.

In the above definitions, by halogen, an atom of fluorine, chlorine or bromine is preferably meant.

The benzoylureas of the present invention can have two different stereoisomer forms: the syn- and the anti-isomer, generally occurring as a mixture with variable composition of both such configurations. The activity of the benzoylureas of the present invention can vary as a function of their stereoisomeric configuration. It is furthermore possible to separate the individual stereoisomers from their mixture, by using known techniques, such as recrystallization and/or chromatography.

The compounds having formula (I) have insecticide and acaricide activity, and are suitable to be used in the agrarian, civil, forestal and veterinary fields, in combating infestations by insects and acari.

In the following description of the preparation of compounds having formula (I), the symbols R, $R_1$, $R_2$, $R_3$, $R_4$, $R_5$, $R_6$ and $R_7$, have the meanings as specified in formula (I), if not differently specified.

The compounds having formula (I) may be obtained by reacting a benzoyl-isocyanate having formula:

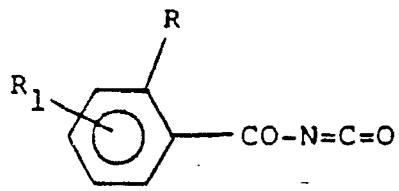

(II)

with an aromatic amine having formula:

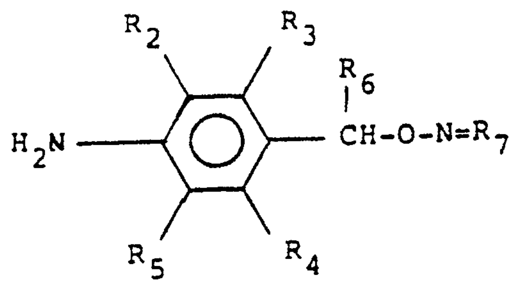

(III)

The reaction does not require the presence of a catalyst and is carried out in an inert solvent, and at a temperature comprised within the range of from 0 ° C to the boiling temperature of the mixture.

Suitable solvents are aromatic hydrocarbons, chlorinated hydrocarbons, ethers, ketones and acetonitrile.

The benzoyl-isocyanates of formula (II) are known compounds, and in some cases they are commercially available.

The amines of formula (III) can be prepared by reducing, by known techniques, nitroderivatives having the formula:

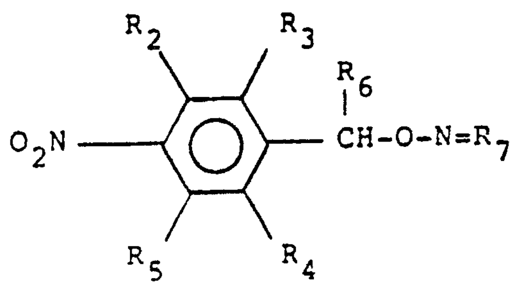

(IV)

In their turn, the nitroderivatives of formula (IV) can be prepared, for example, by reacting a benzyl bromide of formula:

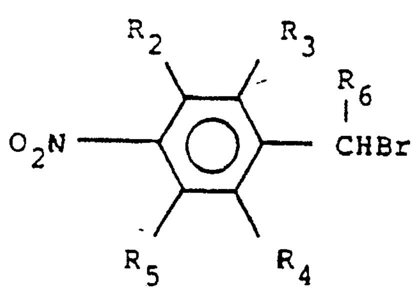

(V)

with the sodium salt of an oxime having formula:

$R_7=N\text{-}OH$ (VI)

The reaction is carried out in dipolar aprotic solvents, using sodium hydride as the base, at a temperature comprised within the range of from 0°C to the boiling temperature of the reaction mixture. The oximes having formula (VI) can require a specific preparation, and in general they can be prepared by known methods, described, for example, in "Organic Functional Group Preparations", Vol. 3, 372-381 (1972). Academic Press, New York.

As will be evident to those skilled in the art, different alternative procedures are available for the synthesis of the intermediates and of the products having formula (I).

An alternative procedure for the synthesis of the compounds of formula (I) comprises reacting a benzamide having the formula:

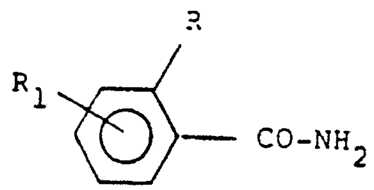

(VII)

with an isocyanate having the formula:

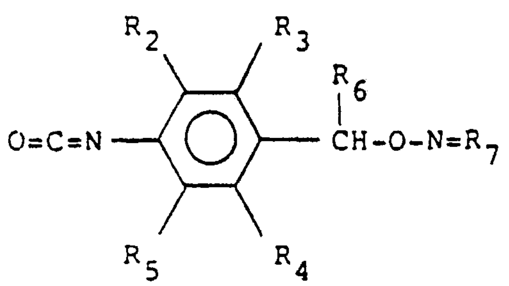

(VIII)

Such a reaction is carried out under conditions similar to those as described above for the reaction between the benzoyl-isocyanate having formula (II) and the amine having formula (III).

However, the preparation of the isocyanates of formula (VIII) involves the preparation of the amines having formula (III) and their reaction with phosgene.

This aspect, together with the fact that the benzoyl-isocyanates having formula (II) are as available as the amides having formula (VII), leads to a general preference for the previously mentioned synthesis method, viz. the reaction between the compounds having formula (II) and (III).

As has been previously mentioned, the compounds of formula (I) have high acaricide and insecticide activity, being particularly active against larvae and eggs of insects and acari.

Among these, in particular, by the compounds having formula (I), the insects belonging to the Lepidoptera, Diptera, Coleoptera and Acari orders can be particularly fought.

These orders comprise numerous species important for their noxiousness in the agrarian, forestal, civil and veterinary fields. Thus, the compounds having formula (I) are suitable for many uses, such as the protection of crops, of forests and of water surfaces against infestations by insects, the protection of stored cereals, and in the veterinary field the protection of breeding cattle against some cattle parasites.

For practical use, the compounds having general formula (I) can be used as such or, more suitably, as compositions containing, besides one or more of compounds having formula (I) as the active ingredient, also solid or liquid inert carriers and, optionally, one or more further additives. According to the usual formulative practice, the compositions can be prepared as aqueous solutions or dispersions, solutions or dispersions in oils, solutions in organic solvents, pastes, wettable powders, dispersable powders, emulsifiable oils, granules, inert emulsions, aerosols and fumigating candles.

The amount of active ingredient in the compositions may vary within a wide range (1-95% by weight), depending on the type of composition and on the use it is intended for.

Should particular situations so require, or for the purpose of extending the activity spectrum, there may be added to the compositions other active substances, such as other insecticides or acaricides.

The amount of active substance (compound having formula I) to be distributed for the insecticide and acaricide treatments depends on various factors such as the type and level of infestation, the environment

wherein the infestation is occurring (agrarian cultivations, water pools or watercourses, organic substrates of various kinds), the type of composition used, climatic and environmental factors, available application means, and so forth. In general, amounts of active substance ranging from 0.01 to 1 kg/ha are sufficient for a good disinfestation.

The invention will be further described with reference to the following illustrative Examples.

In the spectra of proton nuclear magnetic resonance ($^1$H-NMR) reported in the Examples, the following abbreviations are used:

s = singlet:
m = multiplet;
b = (broad) = broad signal;
ABq = quartet of AB type.

Example 1

Preparation of N-(2-chlorobenzyl)-N'-4-[(2-isopropyl-5-methyl)-cyclohexylimino-oxymethyl]-phenyl urea (compound No. 1)

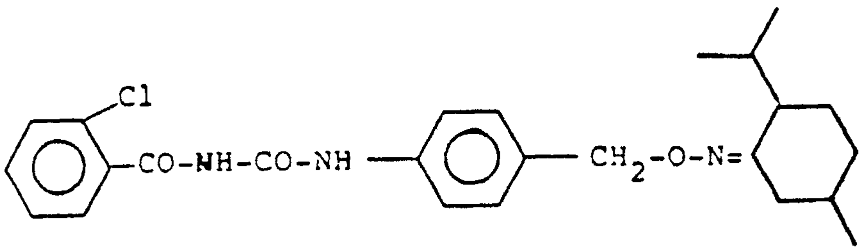

Into a 2-necked 50-ml flask, equipped with a condenser, dropping funnel and magnetic stirrer, 1.0 g of 4-[(2-isopropyl-5-methyl)-cyclohexylimino-oxymethyl]-aniline (prepared as in subsequent Example 3), dissolved in 15 ml of anhydrous ethyl ether, was introduced under nitrogen.

0.7 g of 2-chlorobenzoyl-isocyanate dissolved in 5 ml of anhydrous ethyl ether was then added dropwise at room temperature. The mixture was kept under stirring for 30 minutes, a portion of ether was evaporated under nitrogen, and the residual mass was diluted with hexane, with cooling at 0° C.

The reaction mixture was filtered under nitroen, and the precipitate was washed with hexane and finally dried under nitrogen. 1.0 g of benzoylurea having a melting point (m.p.) of 78-80° C was obtained.

Example 2

Starting from the anilines described in following Example 3, and by operating under conditions similar to those described in Example 1, using 2-chlorobenzoyl-isooyanate, the following compounds were prepared:

- Compound No. 2: N-(2-chlorobenzoyl)-N'-4-[1,7,7-trimethylbicyclo-[2,2,1]-heptane-2-imino-oxymethyl]-phenyl urea

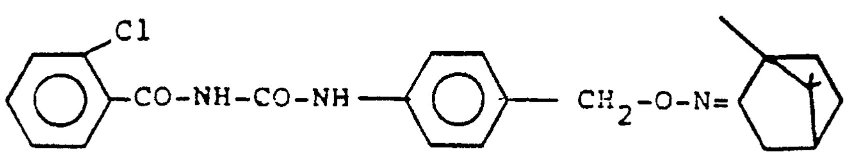

m.p. = 94-96° C

- Compound No. 3: N-(2-chlorobenzoyl)-N'-4-[thiabicyclo-[2,2,2]-oct-5-ene-3-imino-oxymethyl]-phenyl urea

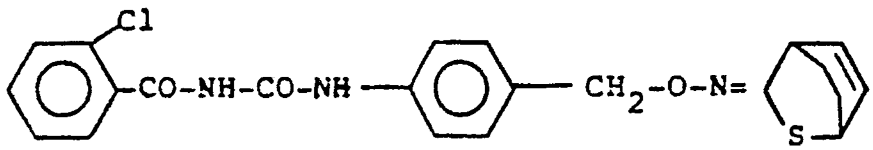

m.p. = 110°C;

while by using 2,6-difluorobenzoyl-isocyanate, the following compounds were prepared:

- Compound No. 4: N-(2,6-difluorobenzoyl)-N'-4-[(2-isopropyl-5-methyl)-cyclohexylimino-oxymethyl]-phenyl urea

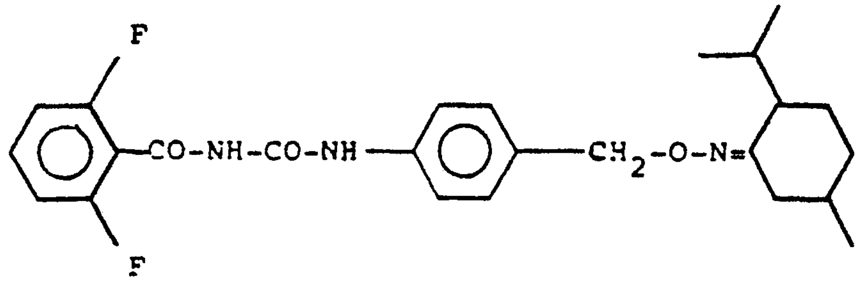

m.p. = 111°C

- Compound No. 5: N-(2,6-difluorobenzoyl)-N'-4-[1,7,7-trimethylbicyclo-[2,2,1]-heptane-2-imino-oxymethyl]-phenyl urea

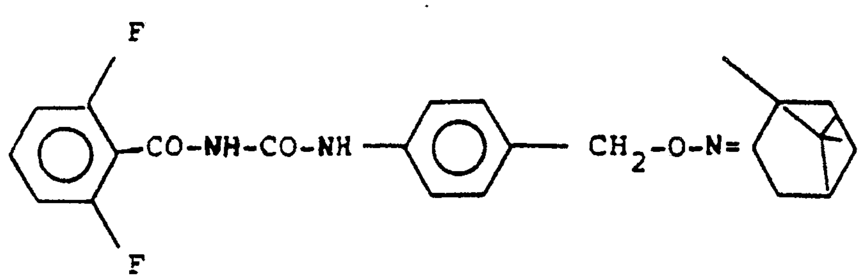

m.p. = 150°C

- Compound No. 6: N-(2,6-difluorobenzoyl)-N'-3,5-dichloro-4-[(2-isopropyl-5-methyl)-cyclohexylimino-oxymethyl]-phenyl urea

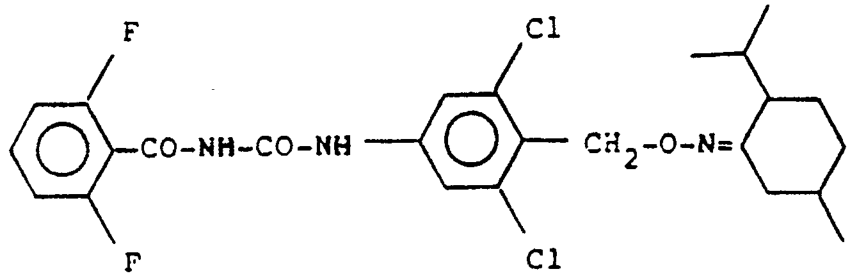

m.p. - 156°C

- Compound No. 7: N-(2,6-difluorobenzoyl)-N'-4-[2-thiabicyclo-[2,2,2]-oct-5-ene-3-imino-oxymethyl]-phenyl urea

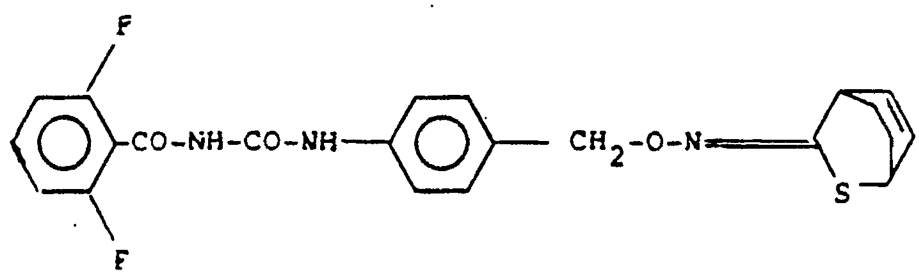

m.p. = 182° C.

- Compound No. 8: N-(2-chlorobenzoyl)-N'-[4-(cyclohexylimino-oxymethyl)phenyl]urea

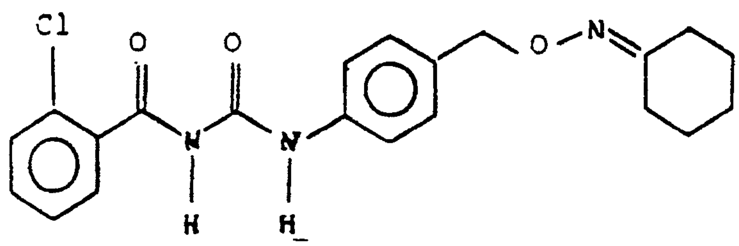

m.p. = 90° C.

- Compound No. 9: N-(2-chlorobenzoyl)-N'-[4-(3,3-dimethyl-1,4-dithian-2-imino-oxymethylphenyl]urea

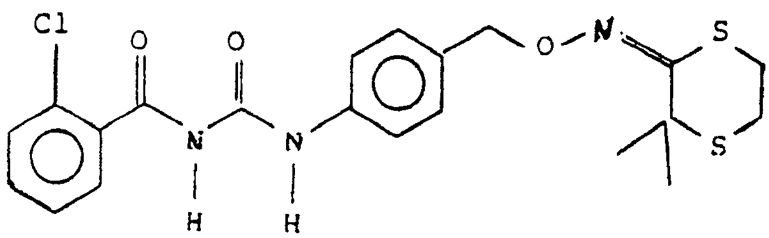

m.p. = 160° C.

- Compound No. 10: N-(2-chlorobenzoyl)-N'-[4-[(2-methyl-5-isopropyl)cyclohexylimino-oxymethyl]-phenyl]-urea

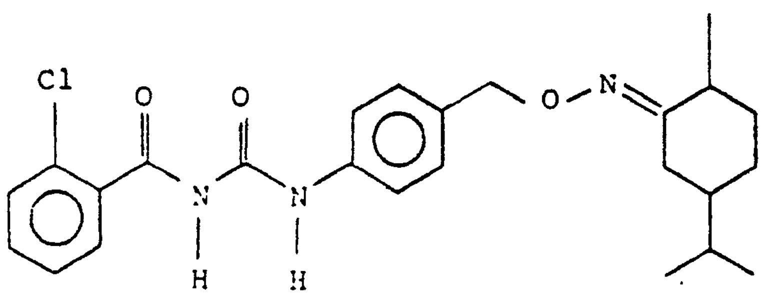

liquid at room temperature.

- Compound No. 11: N-(2-chlorobenzoyl)-N'-[4-[(2-methyl-5-isopropenyl)2-cyclohexenylimino-oxymethyl]-phenyl]urea

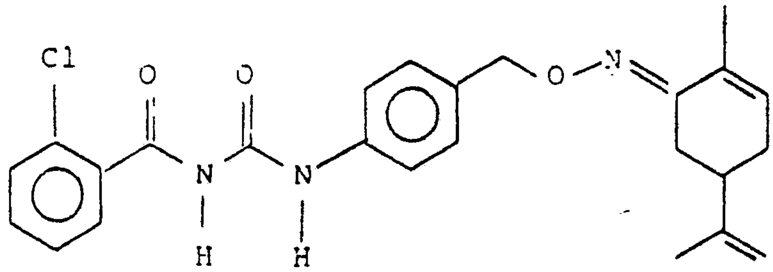

m.p. 145° C.

- Compound No. 12: N-(2-chlorobenzoyl)-N'-[4-[(1-decahydronaphthaleneimino-oxymethyl]phenyl]urea

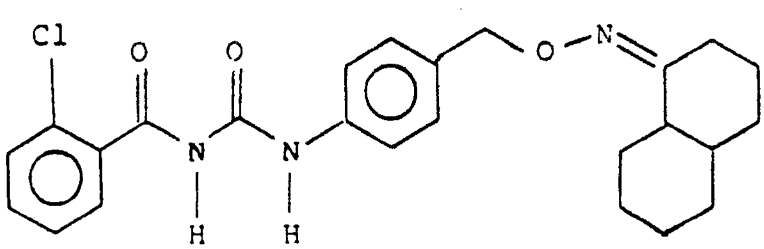

m.p. 129-131° C.

- Compound No. 13: N-(2,6-difluorobenzoyl)-N'-[4-(3,3-dimethyl-1,4-dithian-2-imino-oxymethylphenyl]-urea

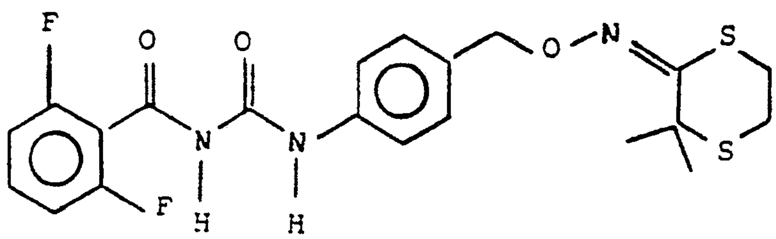

m.p. = 148° C.

- Compound No. 14: N-(2,6-difluorobenzoyl)-N'-[4-[(2-methyl-5-isopropenyl)-2-cyclohexenylimino-oxymethyl]-phenyl]urea

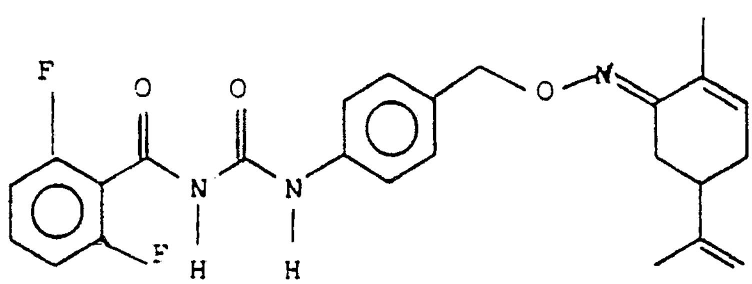

m.p. = 130° C.

- Compound No. 15: N-(2,6-difluorobenzoyl)-N'-[4-cyclohexylimino-oxymethyl)phenyl]urea

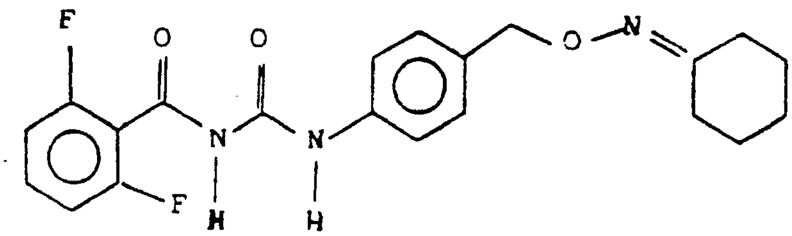

m.p. - 98° C.

- Compound No. 16: N-(2,6-difluorobenzoyl)-N'-[4-[2-tert.butyl-cyclohexylimino-oxymethyl]phenyl]urea

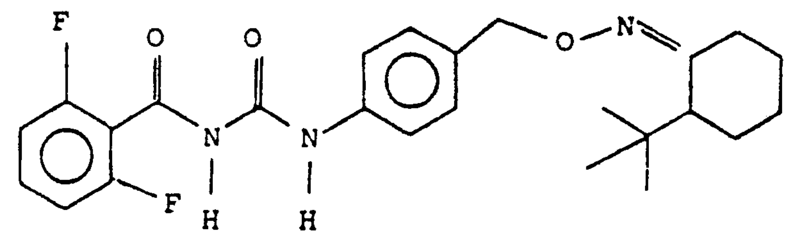

m.p. = 130-133° C.

- Compound No. 17: N-(2,6-difluorobenzoyl)-N'-[4-[1-decahydronaphthaleneimino-oxymethyl]phenyl]-urea

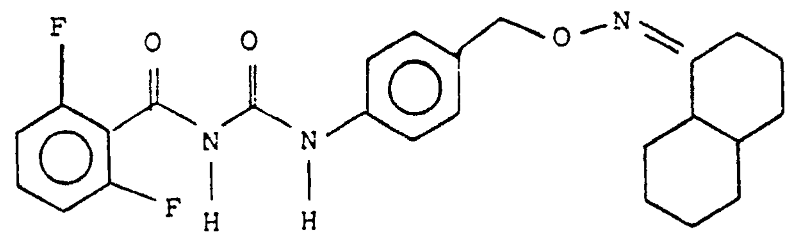

m.p. = 168-171° C.

- Compound No. 18: N-(2,6-difluorobenzoyl)-N'-[4-[(2-methyl-5-isopropyl)cyclohexylimino-oxymethyl]-phenyl]-urea

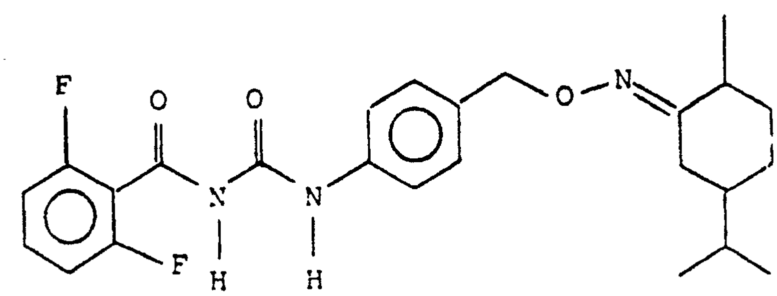

m.p. = 85° C.

Example 3

Preparation of the intermediate anilines

Into a 3-necked flask equipped with a thermometer, condenser and mechanical stirrer, and under nitrogen, 2.2g of iron powder, 8 ml of (95%) ethanol, 2 ml of water, 0.25 ml of hydrochloric acid at 37% and then 2 g of 4-[(2-isopropyl-5-methyl)-cyclohexylimino-oxymethyl]-nitrobenzene (prepared as in following Example 4) dissolved in 2 ml of ethyl alcohol were dissolved in the order as listed. The temperature was kept controlled between 40°C and 50°C for 2 hours. Unreacted iron was filtered off, and the filtrate was diluted with 50 ml of water. The mass was extracted with ethyl ether, and dehydrated with sodium sulphate, and the solvent was removed under reduced pressure. 1.5 g of 4-[(2-isopropyl-5-methyl)-cyclohexylimino-oxymethyl]-aniline was recovered.

$^1$H-NMR ($CDCl_3$): 7.3-6.6 (ABq, 4H, arom.); 4.9 (s, 2H, $-CH_2O$); 3.5 (bs, 2H, $-NH_2$); 3.0-1.1 (m, 9H, aliph.); 1.0-0.8 (m, 9H, $-CH_3$).

By operating under conditions similar to those as described above, and by starting from 4-[1,7,7-trimethyl-bicyclo-[2,2,1]-heptane-2-imino-oxymethyl]-nitrobenzene, 4-[1,7,7-trimethylbicyclo-[2,2,1]-heptane-2-imino-oxymethyl]-aniline was obtained.

$^1$H-NMR ($CDCl_3$): 7.20-6.45 (ABq, 4H, arom.); 4.9 (s, 2H, $-OCH_2-$); 3.65 (s, 2H, $-NH_2$); 2.8-1.05 (m, 7H, aliph.); 1.0-0.6 (m, 9H, $-CH_3$).

By starting from 3,5-dichloro-4-[(2-isopropyl-5-methyl)-cyclo-hexylimino-oxymethyl]-nitrobenzene, there was obtained 3,5-dichloro-4-[(2-isopropyl-5-methyl)-cyclo-hexylimino-oxymethyl]-aniline.

$^1$H-NMR ($CDCl_3$): 6.4 (s, 2H, arom.); 5.0 (s, 2H, $-OCH_2-$); 3.85 (bs, 2H, $-NH_2$); 3.3-0.9 (m, 9H, aliph.); 0.8-0.6 (m, 9H, $-CH_3$).

By starting from 4-[2-thiabicyclo[2,2,2]oct-5-ene-3-imino-oxymethyl]nitrobenzene, there was obtained 4-[2-thiabicyclo-[2,2,2]-oct-5-ene-3-imino-oxymethyl]-aniline.

$^1$H-NMR ($CDCl_3$): 7.4-6.7 (ABq, 4H, arom.): 6.7-6.2 (m, 2H, -CH=CH-): 5.05 (s, 2H, $-OCH_2-$); 4.5 (bs, 2H, $-NH_2$); 4.05-3.70 (m, 2H, -CH-); 2.2-1.5 (m, 4H, $-CH_2$).

By starting from 4-[cyclohexylimino-oxymethyl]nitrobenzene, there was prepared 4-[cyclohexylimino-oxymethyl]aniline.

$^1$H-NMR ($CDCl_3$): 7.2-6.4 (ABq, 4H, arom.); 4.9 (s, 2H, $-CH_2O-$); 3.4 (s, 2H, $-NH_2$); 2.5-1.3 (m, 10H, aliph).

By starting from 4-[3,3-dimethyl-1,4-dithian-2-imino-oxymethyl]nitrobenzene, there was prepared 4-[3,3-dimethyl-1,4-dithian-2-imino-oxymethyl]aniline.

$^1$H-NMR ($CDCl_3$): 7.3-5.5 (ABq, 4H, arom.); 5.0 (s, 2H, $-CH_2O-$); 3.6-3.0 (m, 4H. $-CH_2-$): 3.3 (s, 2H, $-NH_2$); 1.5 (s, 6H, $-CH_3$).

By starting from 4-[(2-methyl-5-isopropyl)cyclohexylimino-oxymethyl]nitrobenzene, there was prepared 4-[(2-methyl-5-isopropyl)cyclohexylimino-oxymethyl]-aniline.

$^1$H-NMR ($CDCl_3$): 7.3-6.6 (ABq, 4H, arom.); 4.9 (s, 2H, $-CH_2O-$); 3.5 (bs, 2H, $-NH_2$); 3.3-0.8 (m, 18H, aliph.).

By starting from 4-[(2-methyl-5-isopropenyl)-2-cyclohexenylimino-oxymethyl]nitrobenzene, there was prepared 4-[(2-methyl-5-isopropenyl)-2-cyclohexenylimino-oxymethyl]aniline.

$^1$H-NMR ($CDCl_3$): 7.3-6.6 (ABq, 4H, arom.); 6.0 (bs, 1H, -CH=C); 5.1 (s, 2H, $-CH_2O-$); 4.8 (s, 2H, $>C=CH_2$); 3.6 (s, 2H, $-NH_2$): 3.3-2.2 (m, 5H, aliph.); 1.9 (s, 3H, $-CH_3$); 1.7 (s, 3H, $-CH_3$).

By starting from 4-[1-decahydronaphthaleneimino-oxymethyl]nitrobenzene, there was prepared 4-[1-decahydronaphthaleneimino-oxymethyl]aniline.

$^1$H-NMR ($CDCl_3$): 7.2-5.5 (ABq, 4H, arom.); 4.9 (s, 2H, $-CH_2O-$); 3.4 (bs, 2H, $-NH_2$); 2.0-1.0 (m, 16H, aliph.).

By starting from 4-[2-tert.butyl-cyclohexylimino-oxymethyl]nitrobenzene, there was prepared 4-[2-tert.butylcyclohexylimino-oxymethyl]aniline.

$^1$H-NMR ($CDCl_3$): 7.2-6.5 (ABq, 4H, arom.); 4.9 (s, 2H, $-CH_2O-$); 3.5 (bs, 2H, $-NH_2$); 3.5-1.5 (m, 9H, aliph.): 1.1 (s, 9H, $-CH_3$).

## Example 4

## Preparation of Intermediate Nitroderivatives

Into a 2-necked 250-ml flask equipped with a condenser, dropping funnel and magnetic stirrer, 100 ml of anhydrous tetrahydrofuran was introduced. Under nitrogen, 1.0 g of sodium hydride was added, and then 4.0 g of 2-isopropyl-5-methyl-cyclohexane-1-one-oxime dissolved in 15 ml of anhydrous tetrahydrofuran was added dropwise. When the evolution of hydrogen had ended, 5.0 g of 4-nitrobenzyl bromide dissolved in 20 ml of anhydrous tetrahydrofuran was added dropwise.

The reaction mass was then kept under stirring at room temperature for 4 hours, filtered, most of the solvent evaporated off, the residue diluted with water, and the whole extracted with ether. The reaction mixture was chromatographed over silica, using a 4:1 hexane-ethyl ether mixture as the eluent. After evaporating off the solvent, there was obtained 4.5 g of 4-[2-isopropyl-5-methyl)-cyclohexylimino-oxymethyl]-nitrobenzene.

$^1H$-NMR ($CDCl_3$): 8.3-7.4 (ABq, 4H, arom.); 5.2 (s, 2H, $-OCH_2-$); 3.1-1.2 (m, 9H, aliph.); 1.0-0.8 (m. 9H, $-CH_3$).

By operating under conditions as described above, by starting from 1,7,7-trimethylbicyclo-[2,2,1]-heptane-2-one-oxime and 4-nitrobenzyl bromide, there was obtained 4-[1,7,7-trimethylbicyclo-[2,2,1]-heptane-2-imino-oxymethyl]-nitrobenzene.

$^1H$-NMR ($CDCl_3$): 8.35-7.5 (ABq, 4H, arom.); 5.2 (s, 2H, $-OCH_2-$); 2.85-1.3 (m, 7H, aliph.); 1.15-0.85 (m, 9H, $-CH_3$).

By starting from 2-isopropyl-5-methylcyclohexane-1-one-oxime and 2.6-dichloro-4-nitrobenzyl bromide, there was prepared 3,5-dichloro-4-[(2-isopropyl-5-methyl)-cyclohexylimino-oxymethyl]-nitrobenzene.

$^1H$-NMR ($CDCl^3$): 7.5 (s, 2H, arom.); 5.3 (s, 2H, $-OCH_2-$); 3.5-1.2 (m, 9H, aliph.); 1.2-0.9 (m, 9H, $-CH_3$).

By starting from 2-thiabicyclo-[2,2,2]-oct-5-en-3-one-oxime and 4-nitrobenzyl bromide, there was prepared 4-[2-thiabicyclo-[2,2,2]-oct-5-ene-3-imino-oxymethyl]-nitrobenzene.

$^1H$-NMR ($CDCl_3$): 8.4-7.55 (ABq, 2H, arom.); 6.8-6.25 (m, 2H, $-CH=CH-$); 5.2 (s, 2H, $-OCH_2-$); 4.2-3.6 (m, 2H, $-CH-$); 2.3-1.8 (m, 4H, $-CH_2-$).

By starting from cyclohexanone oxime and from 4-nitrobenzyl bromide, there was prepared 4-[cyclohexylimino-oxymethyl]nitrobenzene.

$^1H$-NMR ($CDCl_3$): 8.1-7.3 (ABq, 4H, arom.); 4.9 (s, 2H, $-CH_2O-$); 2.5-1.3 (m, 10H, aliph.).

By starting from 3,3-dimethyl-1,4-dithian-2-one oxime and from 4-nitrobenzyl bromide, there was obtained 4-[3,3-dimethyl-1,4-dithian-2-imino-oxymethyl]nitrobenzene.

$^1H$-NMR ($CDCl_3$): 8.2-7.4 (ABq, 4H, arom.); 5.0 (s, 2H, $-CH_2O-$); 3.6-3.0 (m, 4H, $-CH_2-$); 1.5 (s, 6H, $-CH_3$).

By starting from 2-methyl-5-isopropyl-cyclohexane-1-one oxime and from 4-nitrobenzyl bromide, there was prepared 4-[(2-methyl-5-isopropyl)cyclohexylimino-oxymethyl]nitrobenzene.

$^1H$-NMR ($CDCl_3$): 8.1-7.2 (ABq, 4H, arom.): 4.9 (s, 2H, $-CH_2O-$); 3.3-0.9 (m, 18H, aliph.).

By starting from 2-methyl-5-isopropenyl-cyclohex-2-en-1-one oxime and from 4-nitrobenzyl bromide, there was prepared 4-[(2-methyl-5-isopropenyl)-2-cyclohexenylimino-oxymethyl]nitrobenzene.

$^1H$-NMR ($CDCl_3$): 8.2-7.3 (ABq, 4H, arom.); 6.1 (bs, 1H, $-CH=CH-$); 5.3 (s, 2H, $-CH_2O-$); 4.8 (s, 2H, $>C=CH_2$); 3.3-2.2 (m, 5H, aliph.); 1.9 (s, 3H, $-CH_3$); 1.7 (s, 3H, $-CH_3$).

By starting from decahydronaphthalene-1-one oxime and from 4-nitrobenzyl bromide, there was prepared 4-[1-decahydronaphthaleneimino-oxymethyl]nitrobenzene.

$^1H$-NMR ($CDCl_3$): 8.2-7.2 (ABq, 4H, arom.); 5.1 (s, 2H, $-CH_2O-$); 3.0-1.5 (m, 9H, aliph.); 1.1 (s, 9H, $-CH_3$).

By starting from 2-tert.butyl-cyclohexane-1-one oxime and from 4-nitrobenzyl bromide, there was prepared 4-[2-tert.butyl-cyclohexylimino-oxymethyl]nitrobenzene.

$^1H$-NMR ($CDCl_3$): 8.2-7.4 (ABq, 4H, arom.); 5.1 (s, 2H, $-CH_2O-$); 3.0-1.3 (m, 9H, aliph.); 1.0 (s, 9H, $-CH_3$).

Example 5

Test 1 - Determination of activity against Tetranychus urticae - Activity on acarus eggs.

Small discs prepared from bean leaves were infested with eggs of the acarus, and subsequently treated by spraying with a water-acetone solution of the product under test.

The percentage of un-hatched eggs was evaluated 7 days after the treatment, as compared to that of untreated eggs.

Test 2 - Determination of activity against Leptinotarsa decemlineata

Potato sprouts, treated by dipping into a water-acetone (acetone 10%) dispersion of the product under test were contacted with 5-days old larvae of the insect: the same was done for the comparison sprouts, treated with the water-acetone mixture only.

The sprouts remain turgid for some days, due to the damp absorbent cotton surrounding their base portion; each replication consisted of two sprouts infested by 10 larvae. The whole was confined and kept at $23 \pm 1\,^\circ C$, $70 \pm 5\%$ R.H. (relative humidity) and continuous lighting. After 72 hours, the treated vegetable substrate was replaced; the dead % of the larvae was measured 8 days after the infestation time.

The insecticide and acaricide activity in the tests as above described was expressed according to the following scale of values.

5 = complete activity (98-100% dead)
4 = high activity (89-97% dead)
3 = fair activity (60-79% dead)
2 = sufficient activity (40-59% dead)
1 = poor activity (20-39% dead)

0 = negligible or no activity (0-19% dead).

In the following Tables 1 and 2 the data of the insecticide and acaricide activity at the indicated doses is reported, as expressed by the above scale of values.

Table 1

- Activity against Tetranychus urticae

| Compound No. Dose % = | 0.005 | 0.001 | 0.0005 | 0.0001 |
|---|---|---|---|---|
| 1 | 5 | 5 | 5 | 5 |
| 4 | 5 | 5 | 5 | 5 |
| 10 | | | 5 | 3 |
| 11 | 5 | 5 | 4 | 4 |
| 12 | 4 | | | |
| 14 | 5 | 4 | 4 | 2 |
| 17 | 5 | 5 | 5 | 4 |
| 18 | | | 5 | 4 |

Table 2

- Activity against Leptinotarsa decemlineata

| Compound No. Dose % = | 0.005 | 0.001 | 0.0005 |
|---|---|---|---|
| 1 | 5 | 4 | 4 |
| 4 | 5 | 5 | 4 |
| 14 | 5 | 5 | 4 |
| 18 | 5 | 4 | 4 |

## Claims

1. A compound characterized by having the formula:

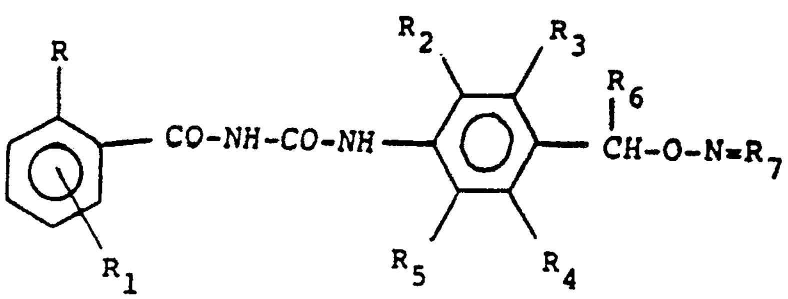

(I)

wherein:

R represents a chlorine or fluorine atom;

$R_1$ represents a hydrogen, chlorine or fluorine atom;

$R_2$ and $R_5$, which may the same as or different from each other, represent a hydrogen or halogen atom, or an alkyl group containing from 1 to 4 carbon atoms;

$R_3$ and $R_4$, which may be the same as or different from each other, represent a hydrogen or halogen atom, or an alkyl, haloalkyl, alkoxy, haloalkoxy, alkenyl, haloalkenyl, alkenyloxy, haloalkenyloxy or alkynyl group;
$R_6$ represents a hydrogen atom or an alkyl or haloalkyl group containing from 1 to 3 carbon atoms; and
$R_7$ represents a monocyclo- bicyclo- or polycyclo-alkyl or -alkenyl group containing from 5 to 10 carbon atoms, or a monocyclo-, bicyclo- or polycyclo-alkyl or -alkenyl group containing from 4 to 8 carbon atoms and 1 or 2 heteroatoms such as N, S, O.

**2.** A compound as claimed in claim 1, which is N-(2-chlorobenzoyl)-N'-4-[(2-isopropyl-5-methyl)-cyclohexylimino-oxymethyl]-phenyl urea.

**3.** A compound as claimed in claim 1, which is N-(2,6-difluorobenzoyl)-N'-4-[(2-isopropyl-5-methyl)-cyclo hexylimino-oxymethyl]-phenyl urea.

**4.** Intermediate compound 4-[(2-isopropyl-5-methyl)-cyclohexylimino-oxymethyl]-aniline.

**5.** Intermediate compound 4-[1,7,7-trimethylbicyclo-[2,2,1]-heptane-2-imino-oxymethyl]-aniline.

**6.** Intermediate compound 3,5-dichloro-4[(2-isopropyl-5-methyl)-cyclohexylimino-oxymethyl]-aniline.

**7.** Intermediate compound 4-[2-thiabicyclo-[2,2,2]-oct-5-ene-3-imino-oxymethyl]-aniline.

**8.** A process for the preparation of a compound according to claim 1, characterized by reacting in an inert solvent, and at a temperature from 0° C to the boiling temperature of the reaction mixture, a benzoyl-isocyanate of formula:

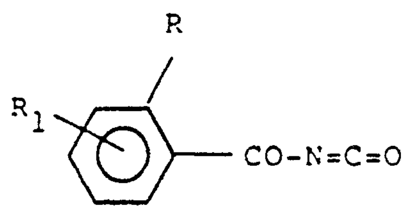

(II)

wherein R and $R_1$ have the same meanings as defined in claim 1, with an aromatic amine having formula:

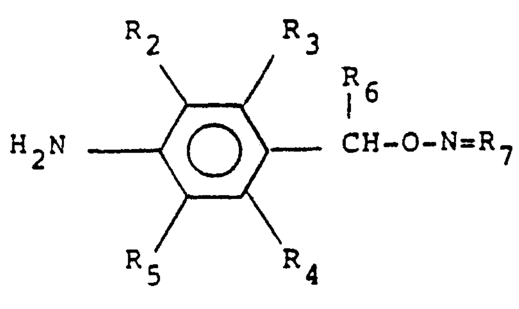

(III)

wherein $R_2$, $R_3$, $R_4$, $R_5$, $R_6$, and $R_7$ have the same meanings as defined in claim 1.

**9.** A method of combating infestations by noxious insects and acari, characterized by applying to the infestation zone an effective amount of one or more compounds as claimed in any of claims 1 to 3, as such or in the form of a suitable composition.

**10.** Acaricide and insecticide compositions containing as their active ingredient one or more compounds as claimed in any of claims 1 to 3, together with a solid or liquid inert carrier and, optionally, one or more other additives.

11. The use of a compound as claimed in any of claims 1 to 3 for combating infestations by noxious insects and acari.

## Revendications

1. Un composé caractérisé en ce qu'il a la formule générale:

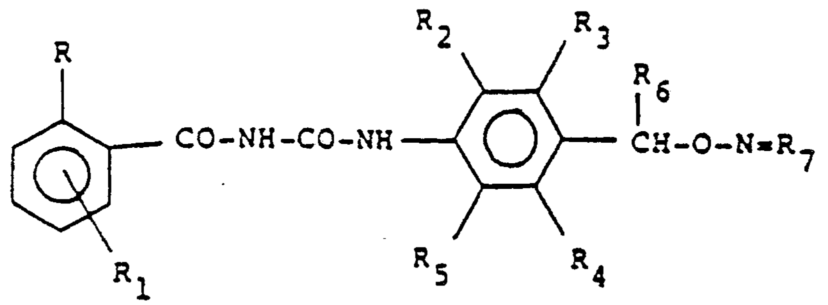

(I)

dans laquelle:

R représente un atome de chlore ou de fluor;

$R_1$ représente un atome d'hydrogène, de chlore ou de fluor;

$R_2$ et $R_5$, qui peuvent être identiques ou différents, représentent chacun un atome d'hydrogène ou d'halogène, ou un groupe alkyle ayant de 1 à 4 atomes de carbone;

$R_3$ et $R_4$, qui peuvent être identiques ou différents, représentent chacun un atome d'hydrogène ou d'halogène, ou un groupe alkyle, haloalkyle, alkoxy, haloalkoxy, alkényle, haloalkényle, alkényloxy, haloalkényloxy, ou alkynyle;

$R_6$ représente un atome d'hydrogène, ou un groupe alkyle ou haloalkyle ayant de 1 à 3 atomes de carbone; et

$R_7$ représente un groupe monocyclo-, bicyclo- ou polycyclo-alkyle ou alkényle ayant de 5 à 10 atomes de carbone, ou un groupe monocyclo-, bicyclo- ou polycyclo-alkyle ou -alkényle, ayant de 4 à 8 atomes de carbone, ayant de 4 à 8 atomes de carbone et 1 ou 2 hétéroatomes, tels que N, S, O.

2. Un composé selon la revendication 1, qui est la N-(2-chlorobenzyl)-N'-4-{(2-isopropyl-5-méthyl)-cyclohexylimino-oxyméthyl}-phénylurée.

3. Un composé selon la revendication 1, qui est la N-(2,6-difluorobenzoyl)-N'-4-{(2-isopropyl-5-méthyl)-cyclohexylimino-oxyméthyl}-phénylurée.

4. Composé intermédiaire 4-{(2-isopropyl-5-méthyl)-cyclohexylimino-oxyméthyl}-aniline.

5. Composé intermédiaire 4-{1,7,7-triméthylbicyclo-{2,2,1}-heptane-2-imino-oxyméthyl)-aniline.

6. Composé intermédiaire 3,5-dichloro-4-{(2-isopropyl-5-méthyl)-cyclohexylimino-oxyméthyl}-aniline.

7. Composé intermédiaire 4-(2-thiabicyclo-(2,2,2}-oct-5-ène-3-imino-oxyméthyl}-aniline.

8. Un procédé pour la préparation d'un composé selon la revendication 1, caractérisé en ce qu'il consiste à faire réagir dans un solvant inerte, et à une température comprise entre 0°C et la température d'ébullition du mélange réactionnel, un isocyanate de benzoyle de formule:

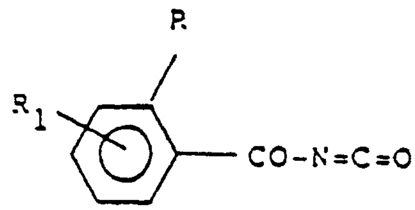

(II)

dans laquelle R et $R_1$ ont les significations données dans la revendication 1, avec une amine aromatique de formule:

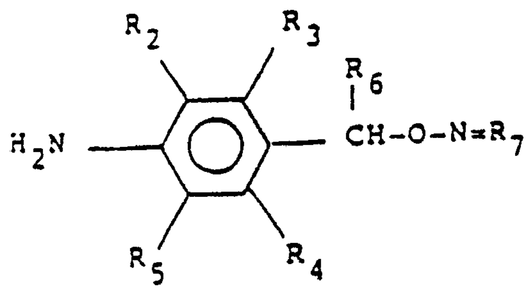

(III)

dans laquelle $R_2$, $R_3$, $R_4$, $R_5$, $R_6$ et $R_7$ ont les significations données dans la revendication 1.

**9.** Un procédé pour combattre les infestations par des insectes et acariens nuisibles, caractérisé en ce qu'il consiste à appliquer sur la zone d'infestation une quantité efficace d'un ou plusieurs composés selon l'une ou plusieurs des revendications 1 à 3, en l'état ou sous la forme d'une composition appropriée.

**10.** Compositions acaricides et insecticides contenant comme matière active un ou plusieurs composés selon l'une quelconque des revendications 1 à 3, avec un support inerte, solide ou liquide, et facultativement un ou plusieurs autres additifs.

**11.** L'utilisation d'un composé selon l'une quelconque des revendications 1 à 3 pour combattre les infestations par des insectes et acariens nuisibles.

**Ansprüche**

**1.** Verbindung, gekennzeichnet durch Formel (I):

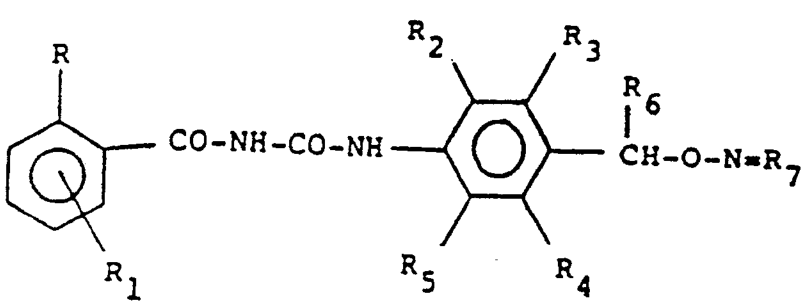

(I)

in der:
R für ein Chlor- oder Fluoratom steht;
$R_1$ für ein Wasserstoff-, Chlor- oder Fluoratom steht;
$R_2$ und $R_5$ gleiche oder unterschiedliche Bedeutungen besitzen und für ein Wasserstoff- oder Halogenatom oder eine Alkylgruppe mit 1 bis 4 Kohlenstoffatomen stehen;

$R_3$ und $R_4$ gleiche oder unterschiedliche Bedeutungen besitzen und für ein Wasserstoff- oder Halogenatom oder eine Alkyl-, Halogenalkyl-, Alkoxy-, Halogenalkoxy, Alkenyl-, Halogen-alkenyl-, Alkenyloxy-, Halogenalkenyloxy- oder Alkinylgruppe stehen;
$R_6$ für ein Wasserstoffatom oder eine Alkyl- oder Halogenalkylgruppe mit 1 bis 3 Kohlenstoffatomen steht;
$R_7$ für eine monocyclische, bicyclische oder polycyclische Alkyl- oder Alkenylgruppe mit 5 bis 10 Kohlenstoffatomen steht, oder für eine monocyclische, bicyclische oder polycyclische Alkyl- oder Alkenylgruppe mit 4 bis 8 Kohlenstoffatomen und 1 oder 2 Heteroatomen, wie N, S, O.

2. Verbindung nach Anspruch 1, dadurch gekennzeichnet, dass es sich um N-(2-Chlorbenzoyl)-N'-4-[(2-isopropyl-5-methyl)-cyclohexylimino-oxymethyl]-phenylharnstoff handelt.

3. Verbindung nach Anspruch 1, dadurch gekennzeichnet, dass es sich um N-(2,6-Difluorbenzoyl)-N'-4- [-(2-isopropyl-5-methyl)-cyclohexylimino-oxymethyl]-phenylharnstoff handelt.

4. Das Zwischenprodukt: 4-[(2-Isopropyl-5-methyl)-cyclohexylimino-oxymethyl]-anilin.

5. Das Zwischenprodukt: 4-[1,7,7-Trimethylbicyclo-[2,2,1]-heptan-2-imino-oxymethyl]-anilin.

6. Das Zwischenprodukt: 3,5-Dichlor-4-[(2-isopropyl-5-methyl)-cyclohexylimino-oxymethyl]-anilin.

7. Das Zwischenprodukt: 4-[2-Thiabicyclo-[2,2,2]-oct-5-en-3-imino-oxymethyl]-anilin.

8. Verfahren zur Herstellung einer Verbindung nach Anspruch 1, dadurch gekennzeichnet, dass in einem inerten Lösungsmittel und bei einer Temperatur zwischen 0 °C und dem Siedepunkt der Reaktionsmischung ein Benzoyl-isocyanat der Formel (II):

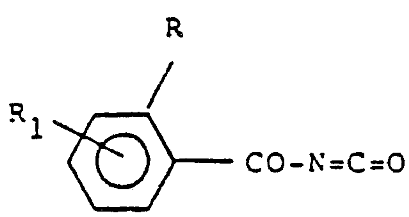

(II)

in der R und $R_1$ die in Anspruch 1 genannten Bedeutungen besitzen, mit einem aromatischen Amin der Formel (III) umgesetzt wird:

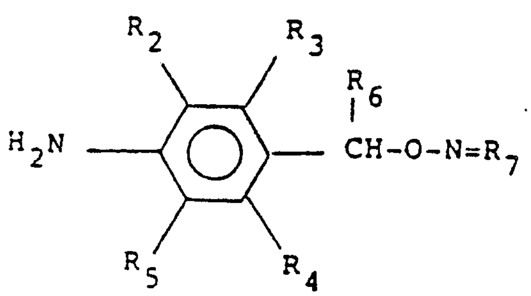

(III)

in der $R_2$, $R_3$, $R_4$, $R_5$, $R_6$ und $R_7$ die in Anspruch 1 genannten Bedeutungen besitzen.

9. Verfahren zur Bekämpfung des Befalls durch schädliche Insekten und Milben, dadurch gekennzeichnet, dass auf die Befallzone eine wirksame Menge einer oder mehrerer Verbindungen gemäss irgendeinem der Ansprüche 1 bis 3 als solche oder in Form einer geeigneten Zusammensetzung aufgebracht wird.

10. Akarizide und insektizide Zusammensetzungen, die als Wirkstoff eine oder mehrere Verbindungen gemäss irgendeinem der Ansprüche 1 bis 3 zusammen mit einem festen oder flüssigen inerten Träger und gegebenenfalls einem oder mehreren anderen Zusatzstoffen enthalten.

11. Verwendung einer Verbindung gemäss irgendeinem der Ansprüche 1 bis 3 zur Bekämpfung des Befalls durch schädliche Insekten und Milben.